(19) Europäisches Patentamt

European Patent Office

Office européen des brevets

(11) Veröffentlichungsnummer: **0 009 694**
**B1**

(12) EUROPÄISCHE PATENTSCHRIFT

(45) Veröffentlichungstag der Patentschrift:
**22.12.82**

(51) Int. Cl.³: **C 07 D 251/34**, C 08 G 18/80,
C 08 G 18/79, C 09 D 3/72

(21) Anmeldenummer: **79103462.2**

(22) Anmeldetag: **17.09.79**

(54) **Lackpolyisocyanat auf Basis von modifiziertem 1-Isocyanato-3,3,5-trimethyl-5-isocyanatomethyl-cyclohexan, ein Verfahren zu seiner Herstellung und seine Verwendung in Zweikomponenten-Polyurethan-Pulverlacken.**

(30) Priorität: **29.09.78 DE 2842641**

(43) Veröffentlichungstag der Anmeldung:
**16.04.80 Patentblatt 80/8**

(45) Bekanntmachung des Hinweises auf die Patenterteilung:
**22.12.82 Patentblatt 82/51**

(84) Benannte Vertragsstaaten:
**AT BE DE FR GB IT NL SE**

(56) Entgegenhaltungen:
**DE-A-2 712 931**
**FR-A-1 455 546**
**FR-A-2 381 810**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **König, Eberhard, Dr., Gelber Weg 22, D-6242 Kronberg (DE)**
Erfinder: **Kreuder, Hans Joachim, Dr., Doerperhofstrasse 35, D-4150 Krefeld 1 (DE)**
Erfinder: **Breidenbach, Peter, Dr., Maarweg 33, D-5000 Köln 41 (DE)**
Erfinder: **Pedain, Josef, Dr., Haferkamp 6, D-5000 Köln 80 (DE)**

Lackpolyisocyanat auf Basis von modifiziertem 1-Isocyanato-3,3,5-trimethyl-5-isocyanatomethyl-cyclohexan, ein Verfahren zu seiner Herstellung und seine Verwendung in Zweikomponenten-Polyurethan-Pulverlacken

Die vorliegende Erfindung betrifft ein neues modifiziertes Isocyanuratgruppen, blockierte Isocyanatgruppen und freie Isocyanatgruppen aufweisendes, festes Lackisocyanat auf Basis von 1-Isocyanato-3,3,5-trimethyl-5-isocyanatomethyl-cy--clohexan (Isophorondiisocyanat, abgekürzt: IPDI), ein Verfahren zu seiner Herstellung durch teilweise Trimerisierung der Isocyanatgruppen von IPDI und anschliessende teilweise Blockierung der verbleibenden Isocyanatgruppen, sowie die Verwendung des neuen modifizierten Polyisocyanats als Vernetzer in Zweikomponenten-Polyurethan-Pulverlacken.

Pulverlacke auf Polyurethan-Basis sind bekannt. Man unterscheidet hierbei Zweikomponenten-Bindemittel (z. B. DE-AS 1 957 483 oder DE-OS 2 105 777) und Einkomponenten-Bindemittel (DE-OS 2 047 718, 2 246 620 und 2 429 517, sowie DE-AS 2 351 477). Obwohl bei den Verfahren dieser Literaturstellen zumindest teilweise aliphatische Diisocyanate zum Einsatz gelangen, ist die Farbstabilität dieser bekannten Systeme oft noch unbefriedigend. Insbesondere neigen sowohl die in DE-OS 2 047 718 und DE-AS 2 351 477 beschriebenen Bindemittel auf Basis von Hexamethylendiisocyanat, die in DE-OS 2 429 517 beschriebenen Bindemittel auf Basis von Toluylendiisocyanat und auch die in DE-OS 2 246 620 und DE-OS 2 105 777 beschriebenen Bindemittel auf Basis von IPDI bzw. von IPDI-Trimethylolpropan-Addukten trotz des mit Ausnahme der Systeme der DE-OS 2 429 517 aliphatischen Charakters der Isocyanatkomponente beim Überbrennen zu starkem Vergilben. Demgegenüber ist von Polyurethanlacken auf Basis von IPDI-Trimerisaten, wie sie beispielsweise in der DE-OS 2 644 684 oder in der deutschen Patentanmeldung P-2 806 731.4 beschrieben sind, bekannt, dass sie besonders inert gegen chemische und physikalische Einflüsse sind und zu Lacken führen, die bezüglich ihrer Wärmefarbstabilität den Lacken der obengenannten Literaturstellen überlegen sind. Die zuletzt genannten IPDI-Trimerisate stellen jedoch Harze mit einer relativ hohen Erweichungstemperatur von ca. 105°C dar. Die Überführung dieser hochschmelzenden Harze in geeignete Pulverlack-Härte durch Blockierung mit z. B. ε-Caprolactam führt deshalb zu erheblichen, technischen Schwierigkeiten. Ausserdem liegt die Erweichungstemperatur des blockierten Harzes weit über den für einen optimalen Verlauf von pulverförmigen Bindemitteln gewünschten Obergrenze von ca. 120–130°C.

Es war daher die Aufgabe der vorliegenden Erfindung, neue, als Vernetzer für Polyurethanpulverlacke geeignete Polyisocyanate zur Verfügung zu stellen, die nicht mit den genannten Nachteilen behaftet sind.

Diese Aufgabe konnte mit den nachstehend näher beschriebenen modifizierten Polyisocyanaten auf IPDI-Basis gelöst werden, welche sowohl einen definierten Gehalt an Isocyanuratgruppen als auch an blockierten Isocyanatgruppen als auch an freien Isocyanatgruppen aufweisen. Die erfindungsgemäss aufgefundene Lösung der gestellten Aufgabe war auch im Hinblick auf FR-A 2 381 810 nicht vorhersehbar, da diese Literaturstelle lediglich blockierte und freie Isocyanatgruppen aufweisende Trimerisate von Diisocyanatotoluol mit einem Gehalt an freien Isocyanatgruppen von 2% beschreibt. Dieser Offenbarung kann jedoch kein Hinweis auf die nachstehend näher beschriebenen erfindungsgemässen Lackpolyisocyanate entnommen werden, da das gemäss Vorveröffentlichung als Ausgangsmaterial eingesetzte Diisocyanatotoluol weder chemisch noch lacktechnisch mit dem erfindungsgemässen Ausgangsmaterial vergleichbar ist. Auch die DE-A 2 712 931 vermittelt keinerlei Hinweis auf die erfindungsgemässen Lackpolyisocyanate, da sich diese Literaturstelle, wie den Ausführungsbeispielen zu entnehmen ist, ausschliesslich mit der Herstellung von total blockierten Polyisocyanaten auf Basis von IPDI befasst.

Gegenstand der vorliegenden Erfindung ist ein Lackpolyisocyanat auf Basis von modifiziertem 1-Isocyanato-3,3,5-trimethyl-5-isocyanato-methylcyclohexan, gekennzeichnet durch einen Gehalt, bezogen auf das Gewicht ohne Einbeziehung des Gewichts des Blockierungsmittels,

a) an trimerisierten, in Form von Isocyanuratgruppen vorliegenden Isocyanatgruppen von 9,45 bis 13,2 Gew.-%,

b) an mit an sich bekannten Blockierungsmitteln blockierten Isocyanatgruppen von 11,3 bis 20,8 Gew.-% und

c) an freien Isocyanatgruppen von 3,8 bis 13,2 Gew.-%.

Gegenstand der Erfindung ist auch ein Verfahren zur Herstellung eines derartigen Lackpolyisocyanats, dadurch gekennzeichnet, dass man entweder in einem ersten Reaktionsschritt 9,45–13,2 Gew.-% der insgesamt 37,8 Gew.-% Isocyanatgruppen des 1-Isocyanato-3,3,5-trimethyl-5-isocyanato-methyl-cyclohexans durch an sich bekannte katalytische Trimerisierung in Isocyanuratgruppen überführt und in einem zweiten Reaktionsschritt weitere 11,3–20,8 Gew.-% der insgesamt im Ausgangsdiisocyanat vorliegenden 37,8 Gew.-% Isocyanatgruppen durch Zugabe einer entsprechenden Menge eines an sich bekannten Blockierungsmittels blockiert, oder man die genannten Reaktionsschritte in umgekehrter Reihenfolge durchführt, so dass von den ursprünglichen 37,8 Gew.-% noch 3,8 bis 13,2 Gew.-%, bezogen auf das Gewicht des modifizierten Polyisocyanats ohne Einbeziehung des Gewichts des vorliegenden Blockierungsmittels, an freien Isocyanatgruppen vorliegen.

Gegenstand der vorliegenden Erfindung ist schliesslich auch die Verwendung der neuen modifizierten Polyisocyanate als Isocyanat-Komponente in Hitze-vernetzbaren Zweikomponenten-Polyurethan-Pulverlacken.

Die erste Stufe des erfindungsgemässen Verfahrens besteht in einer Trimerisierung eines Teils der Isocyanatgruppen des IPDI. Diese Trimerisierung erfolgt in an sich bekannter Weise unter Verwendung von an sich bekannten Trimerisierungskatalysatoren, beispielsweise unter Verwendung von Phosphinen, Alkaliphenolaten, Mischungen aus N,N'-Endoäthylenpiperazin und Propylenoxid, wie sie z.B. in DE-OS 1 934 763, GB-PS 1 392 066, GB-PS 1 386 399 oder DE-OS 2 644 684 beschrieben sind. Zu den besonders bevorzugten Katalysatoren gehören die quaternären, mindestens eine Hydroxyalkyl-Gruppe aufweisenden Ammoniumhydroxide gemäss DE-OS 2 806 731.4, beispielsweise das 2-Hydroxyäthyl-trimethylammoniumhydroxid.

Voraussetzung für die Eignung der Trimerisierungskatalysatoren ist deren Desaktivierbarkeit beispielsweise mittels eines Katalysatorengifts oder durch den Einfluss von Wärme. Die Katalysatoren der DE-OS 2 806 731 weisen den Vorteil auf, dass sie ihre Wirksamkeit sofort, d.h. ohne Inkubationszeit entfalten. Gleichzeitig beginnen sich diese Katalysatoren bei der Temperatur, bei welcher die Trimerisierung stattfindet, thermisch zu zersetzen, so dass der Trimerisierungsgrad bei vorgewählter Ausgangstemperatur auf einfache Weise durch die Menge an zugesetztem Katalysator gesteuert werden kann. Im Falle der Verwendung von thermisch stabilen Katalysatoren ist der Abbruch der Trimerisierungsreaktion beim gewünschten Trimerisierungsgrad (Prozentsatz der trimerisierten Isocyanatgruppen bezogen auf Gesamtmenge der ursprünglich vorliegenden Isocyanatgruppen) durch Zugabe eines geeigneten Katalysatorengifts erforderlich.

Die Trimerisierungsreaktion wird in der ersten Stufe des erfindungsgemässen Verfahrens bei einem NCO-Gehalt des Reaktionsgemischs (bezogen auf Isocyanat-Komponente) von 28,35 bis 24,6 Gew.-% abgebrochen. In der Praxis kann man dabei beispielsweise so verfahren, dass man IPDI in einem Reaktor auf eine Temperatur im Bereich von 40 bis 70 °C erwärmt und den Katalysator (beispielsweise eine Lösung von 2-Hydroxyäthyl-trimethylammoniumhydroxid gemäss nachstehenden Ausführungsbeispielen) so zudosiert, dass die Temperatur bei 80–90 °C gehalten wird. Hierbei sinkt der NCO-Gehalt ausgehend vom Ausgangswert von 37,8 Gew.-% kontinuierlich ab.

Der Trimerisierungsgrad kann hierbei entweder durch die Verwendung einer vorausberechneten bzw. in einem Vorversuch ermittelten Gesamtmenge des Katalysators oder aber auch durch Zugabe eines Katalysatorgifts wie beispielsweise Perfluorbutansulfonsäure eingestellt werden.

Im Anschluss an diese teilweise Trimerisierung der Isocyanatgruppen des Ausgangs-Diisocyanats erfolgt in der zweiten Stufe des erfindungsgemässen Verfahrens die Blockierung eines weiteren Teils der Isocyanatgruppen durch Zugabe eines Blockierungsmittels bei beispielsweise 100 bis 130 °C. Die Menge des Blockierungsmittels wird hierbei so gemessen, dass für jedes Mol an als Ausgangsmaterial eingesetztem IPDI 0,6 bis 1,1 Äquivalente des Blockierungsmittels zur Verfügung stehen, wobei Trimerisierungs- und Blockierungsgrad so gewählt werden, dass mindestens 0,2 und höchstens 0,7 Äquivalente an freien Isocyanatgruppen pro Mol Ausgangsdiisocyanat vorliegen.

Geeignete Blockierungsmittel sind beispielsweise Phenole wie Phenol, die isomeren Kresole, Methyläthyl-ketoxim, Cyclohexanonoxim, Alkohole wie Methanol, tert. Butanol oder Cyclohexanol, Malonsäurediäthylester, Acetessigsäureäthylester, die verschiedenen isomeren Triazole und insbesondere ε-Caprolactam.

Beide Stufen des erfindungsgemässen Verfahrens werden vorzugsweise in Substanz, d.h., von den gegebenenfalls mitverwendeten geringen Lösungsmittelmengen der Katalysatorlösungen abgesehen, lösungsmittelfrei durchgeführt.

Bei der Herstellung der teilblockierten Polyisocyanate ist es zweckmässig, in der angegebenen Reihenfolge zu verfahren, d.h. in der ersten Reaktionsstufe die Cyclopolymerisationsreaktion und erst in der zweiten Reaktionsstufe den Blockierungsschritt durchzuführen. Diese Verfahrensweise hat den Vorteil, dass man den ersten Reaktionsschritt in einem niedrigen Viskositätsbereich durchführen und demzufolge die empfindlichere Trimerisierung besser beherrschen kann. Grundsätzlich denkbar wäre jedoch auch eine Umkehrung der genannten Reaktionsschritte.

Die erfindungsgemässen Verfahrensprodukte weisen somit noch überschüssige freie Isocyanatgruppen, blockierte Isocyanatgruppen und Isocyanuratgruppen auf. Pro Mol an Ausgangsdiisocyanat liegen in den Verfahrensprodukten 0,5 bis 0,7 Äquivalente Isocyanatgruppen in trimerisierter Form, 0,6 bis 1,1 Äquivalente Isocyanatgruppen in blockierter Form und 0,2 bis 0,7 Äquivalente Isocyanatgruppen in freier Form vor, so dass die Summe der Äquivalente an trimerisierten Isocyanatgruppen und blockierten Isocyanatgruppen vorzugsweise mindestens 1,3 beträgt. Dies entspricht einem Gehalt von 9,5 bis 13,2 Gew.-% an Isocyanatgruppen in trimerisierter Form, von 11,3 bis 20,8 Gew.-% an Isocyanatgruppen in blockierter Form und einem Gehalt an freien Isocyanatgruppen von 3,8 bis 13,2 Gew.-%, bezogen auf das Gewicht des modifizierten Polyisocyanats ohne Einbeziehung des Gewichts des vorliegenden Blockierungsmittels. Falls das Blockierungsmittel in die Berechnung der Prozentsätze einbezogen wird, enthalten die erfindungsgemässen Produkte im allgemeinen zwischen 4,4 und 9,5 Gew.-% an trimerisierten NCO-Gruppen, zwischen 8,0 und 16,3 an blockierten NCO-Gruppen und von 1,6 bis 9,0 Gew.-% an freien NCO-Gruppen.

Die erfindungsgemässen Verfahrensprodukte stellen bei Raumtemperatur stabile, feste Verbindungen dar, die nur einen niedrigen Gehalt an Abspaltern aufweisen.

Durch geeignete Wahl sowohl des Trimerisierungs- als auch des Blockierungsgrades innerhalb der genannten Grenzen und auch durch geeignete Wahl des Blockierungsmittels kann im übrigen der optimale Schmelzbereich eingestellt und darüber hinaus Verlauf und Glanz des Lackes bei der erfindungsgemässen Verwendung der erfindungsgemässen Verfahrensprodukte beeinflusst werden.

Trotz ihres oft noch hohen Gehalts an freien Isocyanatgruppen eignen sich die erfindungsgemässen Verfahrensprodukte hervorragend als Vernetzer in Pulverlacken. Nach dem bekannten Stand der Technik auf dem Gebiet der Herstellung von Pulverlackbindemitteln werden die für eine Vernetzung verantwortlichen NCO-Gruppen nur in vollkommen blockierter Form eingesetzt, um die Gefahr der vorzeitigen Aushärtung, sei es während der Lagerung oder während der homogenen Vermischung mit z.B. OH-haltigen Harzen, Pigmenten usw. im Extruder bei Temperaturen von ca. 100–120 °C, zu vermeiden. Es war daher überraschend und nicht zu erwarten, dass sich die erfindungsgemässen Härter zu lagerstabilen, anwendungsfertigen Lackpulvern verarbeiten lassen.

Die erfindungsgemässen Verfahrensprodukte werden bei ihrer Verwendung als Härter für Pulverlacke mit den üblichen Harzen für Pulverlacke auf Polyurethanbasis, d.h. vorzugsweise mit hydroxylgruppenhaltigen Harzen kombiniert.

Als hydroxylgruppenhaltige Harze eignen sich solche, deren Erweichungstemperaturen – bestimmt nach der Differentialthermoanalyse (DTA) – zwischen etwa 40 und 140 °C, vorzugsweise zwischen etwa 45 und 100 °C liegen, deren Hydroxylzahlen zwischen 30 und 200, vorzugsweise 40 und 130, und deren mittleres Molekulargewicht zwischen 400 und 10 000, vorzugsweise zwischen 1000 und 5000, liegen.

Derartige hydroxylgruppenhaltige Harze sind beispielsweise:

1. Hydroxylgruppenhaltige Polyester, die in üblicher Weise aus aromatischen oder aliphatischen oder cycloaliphatischen Glykolen bzw. Polyolen, vorzugsweise aufgrund der besseren Kreidungs- und Wetterbeständigkeit aus aliphatischen oder cycloaliphatischen Glykolen, und Polycarbonsäuren bzw. deren funktionellen Derivaten (Estern, Säurechloriden usw.) erhalten werden (vgl. Houben-Weyl, XIV/2, S. 1–46). Geeignete Glykole sind z.B. Äthylenglykol, 1,2-Propandiol, 1,4-Butandiol sowie Isomere, Neopentylglykol, 1,6-Hexandiol und Isomere, 4,4'-Dihydroxydicyclohexylpropan-2,2 und Cyclohexandiol. Geeignete Polyole sind z.B. Trimethylpropan und Hexantriol. Geeignete Polycarbonsäuren sind z.B. Tetrahydrophthalsäure, Terephthalsäure, Phthalsäure, Isophthalsäure und Trimellithsäure. Besonders bevorzugt sind hydroxylgruppenhaltige Polyester, die aus etwa 50–65 Gew.-% aromatischen Polycarbonsäuren, 30–45 Gew.-% aliphatischen Glykolen und etwa 5 Gew.-% aliphatischen Triolen aufgebaut sind.

2. Hydroxylgruppenhaltige Copolymerisate, wie sie durch Copolymerisation von z.B. Hydroxyalkylacrylaten bzw. -methacrylaten mit Acryl- bzw. Methacrylsäurealkylestern, sowie gegebenenfalls weiteren olefinisch ungesättigten Monomeren und/oder wie sie gemäss der DE-OS 2 137 239 aus Styrol-Maleinsäure-Copolymerisaten durch teilweise Veresterung der Säuregruppen mit Äthylenoxid erhalten werden.

Zur Herstellung der gebrauchsfertigen Lackpulver werden hydroxylgruppenhaltiges Harz und das erfindungsgemässe Lackpolyisocyanat gemischt und gegebenenfalls mit Pigmenten, wie z.B. Titandioxid, Verlaufmittel, wie z.B. Polybutylacrylat oder Silikonverbindungen und anderen üblichen Zusatzstoffen versehen und auf Extrudern oder Knetern bei Temperaturen zwischen 100 und 120 °C in der Schmelze zu einem homogenen Material vermischt. Der erstarrte Feststoff wird gemahlen und durch Sieben von den Kornanteilen oberhalb 0,1 mm befreit. Die hydroxylgruppenhaltigen Harze und erfindungsgemässen Verfahrensprodukte gelangen hierbei in solchen Mengenverhältnissen zum Einsatz, dass für jede Hydroxylgruppe des hydroxylgruppenhaltigen Harzes insgesamt 0,6 bis 1,2, vorzugsweise 0,8 bis 1,0 freie oder blockierte Isocyanatgruppen zur Verfügung stehen.

Die so hergestellten Pulverlacke können nach üblichen Pulverauftrags-Verfahren, wie z.B. elektrostatisches Pulverversprühen oder Wirbelsintern, auf die zu überziehenden Formteile aufgebracht werden. Die Härtung der Überzüge erfolgt durch Erhitzen auf Temperaturen von 150–220 °C, vorzugsweise 170–190 °C. Man erhält harte, glänzende und elastische Überzüge mit hervorragenden Korrosionsschutzeigenschaften und einer sehr guten Wärmefarbstabilität. Im Vergleich zu vollkommen blockierten Bindemitteln können die erfindungsgemäss hergestellten Pulverlacke bei einer niedrigeren Einbrenntemperatur ausgehärtet werden.

In den nachstehenden Beispielen stellen alle Prozentangaben, mit Ausnahme der Glanzwerte, Gewichtsprozente dar.

Darstellung der 2-Hydroxyäthyl-trimethylammoniumhydroxid-Katalysatorlösung

In eine Mischung aus 59 g (1 Mol) Trimethylamin, 50 g Wasser und 50 g Methanol werden unter Rühren 44 g (1 Mol) Äthylenoxid eingeleitet, wobei die Temperatur während der Umsetzung bei 50 °C gehalten wird. Die dunkel gefärbte Lösung des Reaktionsproduktes wird anschliessend mit 827 g Dimethylformamid, entsprechend einer 10%igen Lösung, verdünnt und ist danach anwendungsfertig.

Darstellung von erfindungsgemässen Polyisocyanat-Härtern (Beispiele 1 bis 3)
Beispiel 1

2664 g (12 Mol) 3-Isocyanato-3,3,5-trimethyl-5-isocyanatomethyl-cyclohexan (IPDI) werden bei 60 °C vorgelegt. Unter Rühren gibt man ca. 20 ml der oben beschriebenen Katalysatorlösung in 2 Portionen hinzu, wobei sofort ein Temperaturanstieg zu beobachten ist. Gegebenenfalls durch Kühlung wird eine Innentemperatur von 80–90 °C gehalten, bis der NCO-Gehalt auf 27,5% nach etwa 30–40 Minuten abgesunken ist. Danach rührt man eine Mischung aus 1 ml Perfluorbutansulfonsäure in 2 ml Dimethylformamid ein, um den basischen Katalysator zu neutralisieren. Die Polyisocyanatmischung, die ca. 55 Gew.-% cyclopolymerisiertes IPDI enthält, entsprechend einer Umsetzung von 0,55 Äquivalenten an Isocyanatgruppen pro Mol an eingesetztem IPDI, und bei 20°C eine Viskosität von etwa 60 000 mPas aufweist, kann gelagert oder wie folgt weiterverarbeitet werden.

Man erhöht die Temperatur auf 90–100°C und lässt unter Rühren 985 g (8,7 Mol) geschmolzenes ε-Caprolactam zulaufen. Die exotherme Blockierungsreaktion lässt die Temperatur des Ansatzes auf 120–130°C ansteigen. Zur Vervollständigung der Umsetzung rührt man noch 30 Minuten bei 130°C nach und giesst die Schmelze aus. Das erstarrte Harz, dessen für die Vernetzungsreaktion noch zur Verfügung stehenden NCO-Gruppen zu etwa 50% blockiert und zu etwa 50% frei vorliegen, hat eine Glasumwandlungstemperatur von 47°C. Der analytisch bestimmte Gehalt des modifizierten IPDI an blockierten NCO-Gruppen (berechnet als NCO) liegt bei 10%, der Gehalt an freien NCO-Gruppen bei 8,5%, so dass sich das NCO-Äquivalentgewicht unter Einbeziehung des Gewichtes des Blockierungsmittels zu 227 g errechnet. Von den ursprünglich eingesetzten 37,8% NCO-Gruppen des IPDI liegen in dem beschriebenen Verfahrensprodukt 10,3% in trimerisierter, 13,7% in blockierter und 11,8% in freier Form vor. Die nicht erfassten NCO-Gruppen (2%) sind unter Allophanatisierung abreagiert.

Beispiel 2

2664 g (12 Mol) IPDI werden, wie in Beispiel 1 beschrieben, bis zu einem NCO-Gehalt von ca. 27,5% trimerisiert. Der Trimerisierungskatalysator wird dann durch 15-minütiges Erhitzen auf 120°C unwirksam gemacht. Anschliessend werden 70% der noch vorhandenen NCO-Gruppen durch Zugabe von 1380 g (12,2 Mol) ε-Caprolactam in der in Beispiel 1 beschriebenen Weise blockiert. Das erstarrte Harz hat eine Glasumwandlungstemperatur von 52°C, einen analytisch bestimmten Gehalt an blockierten NCO-Gruppen (berechnet als NCO) von 12,6% und einen Gehalt an freien NCO-Gruppen von 5,4%, so dass sich ein NCO-Äquivalentgewicht unter Einbeziehung des Gewichts des Blockierungsmittels von 233 g ergibt. Dieses Lackpolyisocyanat enthält 10,3% NCO-Gruppen in trimerisierter, 19,2% NCO-Gruppen in blockierter und 8,2% NCO-Gruppen in freier Form, bezogen auf das Gewicht ohne Einbeziehung des Gewichts des Blockierungsmittels.

Beispiel 3

2664 g (12 Mol) IPDI werden, wie in Beispiel 1 beschrieben, bis zu einem NCO-Gehalt von ca. 24,6% umgesetzt. Dieser NCO-Gehalt entspricht der Cyclopolymerisierung von 0,7 Äquivalenten NCO-Gruppen pro 1 Mol IPDI. Die Viskosität dieser Polyisocyanat-Mischung bei 20°C beträgt 130 000 mPas. Bei 100°C lässt man unter Rühren 900 g (8 Mol) ε-Caprolactam, entsprechend einer ca. 50%igen Blockierung der noch vorliegenden NCO-Gruppen, zulaufen. Die Innentemperatur wird dabei auf 140°C gesteigert und die Umsetzung 20 Minuten bei dieser Temperatur durchgeführt. Die Schmelze wird ausgegossen und das erstarrte Harz zerkleinert. Der Härter weist eine Glasumwandlungstemperatur von 50°C, einen analytisch bestimmten Gehalt an blockierten NCO-Gruppen von 9,1%, einen Gehalt an freien NCO-Gruppen von 8,6% und somit ein NCO-Äquivalentgewicht unter Einbeziehung des Gewichts des Blockierungsmittels von 237 g auf.

Von den ursprünglich eingesetzten 37,8% NCO-Gruppen liegen in diesem Lackpolyisocyanat 13,2% in trimerisierter, 11,8% in blockierter und 11,2% in freier Form vor.

Die nicht erfassten NCO-Gruppen (1,6 Gew.-%) sind offensichtlich unter Allophanatisierung abreagiert.

Verwendungsbeispiele (Beispiele 4 bis 7)

Beispiel 4

49,5 Gewichtsteile eines hydroxylgruppenhaltigen Polyesters auf Basis von Terephthalsäure, Neopentylglykol, Hexandiol-1,6 und Trimethylolpropan, der OH-Zahl 50 und des OH-Äquivalentgewichts 1120, 10,0 Gewichtsteile des Härters gemäss Beispiel 1, 39,9 Gewichtsteile eines handelsüblichen Titanpigments (Rutil) und 0,6 Gewichtsteile eines handelsüblichen Verlaufmittels auf Acrylatbasis werden in einem Extruder bei ca. 120°C aufgeschmolzen und homogenisiert. Die Dosierung des Polyhydroxidpolyesters und des Härters erfolgt hierbei im Verhältnis der Äquivalentgewichte. Nach dem Erstarren der Schmelze wird das Produkt gemahlen. Das Lackpulver weist eine Glasumwandlungstemperatur von 53°C auf. Die Kornfraktion von 0,02–0,1 mm wird mittels einer elektrostatischen Sprüheinrichtung auf Prüfbleche appliziert und im Einbrennofen während 15 Minuten bei 180°C ausgehärtet. Folgende lacktechnische Werte resultieren:

Erichsentiefung > 10 mm
(DIN 53 156)
Erichsentiefung nach 3 Tagen Alterung bei 70°C 10 mm
Glanz-60° Reflexionswinkel (ASTM-D 523) 91%
konischer Dornbiegetest (ASTM 522) 35%
Stufen-Dornbiegetest (DIN 53 152) 2 mm
Gitterschnitt (DIN 53 151) GTO

Die Überbrennfestigkeit dieses Lackes sei anhand der folgenden Testreihe demonstriert:

Hitzebehandlung: 10′ 200°C, 15′ 200°C, 30′ 200°C, 10′ 220°C, 20′ 220°C,

Weissgrad nach A. Berger 81,9/80,8/79,8/79,0/78,0

Beispiel 5

49,3 Gewichtsteile des hydroxylgruppenhaltigen Polyesters aus Beispiel 4, 10,2 Gewichtsteile des Härters aus Beispiel 2, 39,9 Gewichtsteile des Titanpigments aus Beispiel 4 und 0,6 Gewichtsteile des Verlaufmittels aus Beispiel 4 werden wie in Beispiel 4 beschrieben zu einem Pulverlack verarbeitet.

Die Dosierung der Bindemittelkomponenten erfolgt hierbei im Verhältnis des Äquivalentgewichts. Die beschichteten Prüflacke zeigen nach dem Einbrennen 10' bei 200 °C eine ausgezeichnete Kantendeckung sowie eine vollkommen störungsfreie Oberfläche bei einer Schichtdicke von ca. 0,07 mm. Weitere lacktechnische Werte sind:
Erichsentiefung > 10 mm
Glanz-60° 92%
Weissgrad nach A. Berger 82,5
konischer Dornbiegetest 35%
Stufen-Dornbiegetest 2 mm
Gitterschnitt GTO

Beispiel 6

49,1 Gewichtsteile des hydroxylgruppenhaltigen Polyesters aus Beispiel 4, 10,4 Gewichtsteile des Härters aus Beispiel 3, 39,9 Gewichtsteile des Titanpigments aus Beispiel 4 und 0,6 Gewichtsteile des Verlaufmittels aus Beispiel 4 werden zu einem Pulverlack verarbeitet. Die Dosierung der Bindemittelkomponenten erfolgt hierbei im Verhältnis der Äquivalentgewichte. Die beschichteten Prüfbleche werden sowohl 10' bei 200 °C als auch 30' bei 160 °C eingebrannt. Es resultieren folgende Lackeigenschaften:
Erichsentiefung 10 mm
Glanz-60° 91–92%
Weissgrad nach A. Berger 84–86
konischer Dornbiegetest 35%
Stufen-Dornbiegetest 2 mm
Gitterschnitt GTO

Beispiel 7

52,7 Gewichtsteile des hydroxylgruppenhaltigen Polyesters aus Beispiel 4, 6,8 Gewichtsteile des Härters aus Beispiel 3, 39,9 Gewichtsteile des Titanpigments aus Beispiel 4 und 0,6 Gewichtsteile des Verlaufmittels aus Beispiel 4 werden wie in Beispiel 4 zu einem Pulverlack verarbeitet. Entsprechend einer Untervernetzung erfolgt hierbei die Dosierung der Bindemittelkomponenten im Äquivalentverhältnis OH/NCO = 1/0,6. Die beschichteten Prüfbleche haben nach dem Einbrennen 10' bei 200 °C folgende lacktechnische Eigenschaften:
Erichsentiefung 10 mm
Glanz-60° 91,5%
Weissgrad nach A. Berger 85,5
Gitterschnitt GTO
Salzsprühtest 500 Std. ohne Befund
(DIN 53 167)
Schwitzwassertest 100 Std. ohne Befund
(DIN 50 017)
Abriebbeständigkeit 60 mg Abrieb
(Tabor-Abraisor CS 10-Stein, 1000 Umdrehungen)

Die in den Beispielen 4 bis 7 beschriebenen gebrauchsfertigen Pulverlacke sind bei einer Temperatur von 45 bis 50 °C über einen Zeitraum von 4 Wochen rieselfähig. Sie verlieren bei einer Lagerung bei Raumtemperatur über einen Zeitraum von einem halben Jahr die beschriebenen Gebrauchs- und Filmeigenschaften nicht.

**Patentansprüche für die Vertragsstaaten: BE, DE, FR, GB, IT, NL, SE**

1. Lackpolyisocyanat auf Basis von modifiziertem 1-Isocyanato-3,3,5-trimethyl-5-isocyanato-methylcyclohexan, gekennzeichnet durch einen Gehalt, bezogen auf das Gewicht ohne Einbeziehung des Gewichts des Blockierungsmittels,

a) an trimerisierten, in Form von Isocyanuratgruppen vorliegenden Isocyanatgruppen von 9,45 bis 13,2 Gew.-%,

b) an mit an sich bekannten Blockierungsmitteln blockierten Isocyanatgruppen von 11,3 bis 20,8 Gew.-% und

c) an freien Isocyanatgruppen von 3,8 bis 13,2 Gew.-%.

2. Verfahren zur Herstellung eines Lackpolyisocyanats gemäss Anspruch 1, dadurch gekennzeichnet, dass man entweder in einem ersten Reaktionsschritt 9,45–13,2 Gew.-% der insgesamt 37,8 Gew.-% Isocyanatgruppen des 1-Isocyanato-3,3,5-trimethyl-5-isocyanato-methyl-cyclohexans durch an sich bekannte katalytische Trimerisierung in Isocyanuratgruppen überführt und in einem zweiten Reaktionsschritt weitere 11,3–20,8 Gew.-% der insgesamt im Ausgangsdiisocyanat vorliegenden 37,8 Gew.-% Isocyanatgruppen durch Zugabe einer entsprechenden Menge eines an sich bekannten Blockierungsmittels blockiert, oder man die genannten Reaktionsschritte in umgekehrter Reihenfolge durchführt, so dass von den ursprünglichen 37,8 Gew.-% noch 3,8 bis 13,2 Gew.-%, bezogen auf das Gewicht des modifizierten Polyisocyanats ohne Einbeziehung des Gewichts des vorliegenden Blockierungsmittels, an freien Isocyanatgruppen vorliegen.

3. Verwendung des Lackpolyisocyanats gemäss Anspruch 1 als Isocyanatkomponente in Hitzevernetzbaren Zweikomponenten-Polyurethan-Pulverlacken.

**Patentansprüche für den Vertragsstaat: AT**

1. Verfahren zur Herstellung eines Lackpolyisocyanats auf Basis von modifiziertem 1-Isocyanato-3,3,5-trimethyl-5-isocyanato-methyl-cyclohexan, dadurch gekennzeichnet, dass man entweder in einem ersten Reaktionsschritt 9,45–13,2 Gew.-% der insgesamt 37,8 Gew.-% Isocyanatgruppen des 1-Isocyanato-3,3,5-trimethyl-5-isocyanato-methyl-cyclohexans durch an sich bekannte katalytische Trimerisierung in Isocyanuratgruppen überführt und in einem zweiten Reaktionsschritt weitere 11,3–20,8 Gew.-% der insgesamt im Ausgangsdiisocyanat vorliegenden 37,8 Gew.-% Isocyanatgruppen durch Zugabe einer entsprechenden Menge eines an sich bekannten Blockierungsmittels blockiert, oder man die genannten Reaktionsschritte in umgekehrter Reihenfolge durchführt, so dass von den ursprünglichen 37,8 Gew.-% noch 3,8 bis 13,2 Gew.-%, bezogen auf das Gewicht des modifizierten Polyisocyanats ohne Einbeziehung des Gewichts des vorliegenden Blockierungsmittels, an freien Isocyanatgruppen vorliegen.

2. Verwendung des beim Verfahren gemäss Anspruch 1 erhaltenen Lackpolyisocyanats als Isocyanatkomponente in Hitze-vernetzbaren Zweikomponenten-Polyurethan-Pulverlacken.

**Claims for the Contracting States: BE, DE, FR, GB, IT, NL, SE**

1. A lacquer-grade polyisocyanate based on modified 1-isocyanato-3,3,5-trimethyl-5-isocyanatomethyl cyclohexane, characterised by a content, based on the weight excluding the weight of the blocking agent,
a) of 9.45 to 13.2% by weight of trimerised isocyanate groups in the form of isocyanurate groups,
b) of 11.3 to 20.8% by weight of isocyanate groups blocked with blocking agents known per se and
c) of 3.8 to 13.2% by weight of free isocyanate groups.
2. A process for producing the lacquer-grade polyisocyanate according to claim 1, characterised in that either, in a first reaction step, 9.45–13.2% by weight of the total of 37.8% by weight of isocyanate groups in the 1-isocyanato-3,3,5-trimethyl-5-isocyanatomethyl cyclohexane are converted into isocyanurate groups by catalytic trimerisation by a known method and, in a second reaction step, a further 11.3 to 20.8% by weight of the total of 37.8% by weight of isocyanate groups present in the starting diisocyanate are blocked by the addition of a corresponding quantity of a blocking agent known per se, or the indicated reaction steps are carried out in the reverse sequence, such that of the original 37.8% by weight 3.8 to 13.2% by weight, based on the weight of the modified polyisocyanate excluding the weight of the blocking agent present, of free isocyanate groups are still present.
3. The use of the lacquer-grade polyisocyanate according to claim 1, as isocyanate component in heat-crosslinkable two-component polyurethane powder lacquers.

**Claims for the Contracting State: AT**

1. A process for producing a lacquer-grade polyisocyanate based on modified 1-isocyanato-3,3,5-trimethyl-5-isocyanatomethyl cyclohexane, characterised in that either, in a first reaction step, 9.45–13.2% by weight of the total of 37.8% by weight of isocyanate groups in the 1-isocyanato-3,3,5-trimethyl-5-isocyanatomethyl cyclohexane are converted into isocyanurate groups by catalytic trimerisation by a known method and, in a second reaction step, a further 11.3 to 20.8% by weight of the total of 37.8% by weight of isocyanate groups present in the starting diisocyanate are blocked by the addition of a corresponding quantity of a blocking agent known per se, or the indicated reaction steps are carried out in the reverse sequence, such that of the original 37.8% by weight 3.8 to 13.2% by weight, based on the weight of the modified polyisocyanate excluding the weight of the blocking agent present, of free isocyanate groups are still present.

2. The use of the lacquer-grade polyisocyanate obtained by the process according to claim 1, as isocyanate component in heat-crosslinkable two-component polyurethane powder lacquers.

**Revendications pour les Etats contractants: BE, DB, FR, GB, IT, NL, SE**

1. Polyisocyanate pour vernis à base de 1-isocyanato-3,3,5-triméthyl-5-isocyanatométhylcyclohexane modifié, caractérisé en ce qu'il contient, par rapport au poids, compte non tenu du poids de l'agent de blocage,
a) 9,45 à 13,2% en poids de groupes isocyanates trimérisés, sous forme de groupes isocyanurates,
b) 11,3 à 20,8% en poids de groupes isocyanates bloqués par des agents bloquants connus, et
c) 3,8 à 13,2% en poids de groupes isocyanates libres.
2. Procédé pour la fabrication d'un polyisocyanate pour vernis selon la revendication 1, caractérisé en ce que ou bien on transforme dans une première étape de réaction 9,45–13,2% en poids sur le total des 37,8% en poids de groupes isocyanates du 1-isocyanato-3,3,5-triméthyl-5-isocyanatométhylcyclohexane en groupes isocyanurates par trimérisation catalytique connue et on bloque dans une seconde étape de réaction encore 11,3–20,8% en poids sur le total des 37,8% en poids de groupes isocyanates présents dans le diisocyanate de départ par addition d'une quantité correspondante d'un agent bloquant connu, ou bien on effectue dans l'ordre inverse les étapes de réaction mentionnées, de sorte que, sur les 37,8% en poids de départ, il y ait encore 3,8 à 13,2% en poids de groupes isocyanates libres par rapport au poids du polyisocyanate modifié, compte non tenu du poids de l'agent bloquant présent.
3. Utilisation du polyisocyanate selon la revendication 1, comme composant isocyanate dans les vernis en poudre de polyuréthannes à deux composants thermoréticulables.

**Revendications pour l'Etat contractant: AT**

1. Procédé pour la préparation d'un polyisocyanate pour vernis à base de 1-isocyanato-3,3,5-tri- méthyl-5-isocyanato-méthylcyclohexane modifié, caractérisé en ce que ou bien on transforme dans une première étape de réaction 9,45–13,2% en poids sur le total des 37,8% en poids de groupes isocyanates du 1-isocyanato-3,3,5-triméthyl-5-iso-cyanatométhylcyclohexane en groupes isocyanurates par trimérisation catalytique connue et on bloque dans une seconde étape de réaction encore 11,3–20,8% en poids sur le total des 37,8% en poids de groupes isocyanates présents dans le diisocyanate de départ par addition d'une quantité correspondante d'un agent bloquant connu, ou bien on effectue dans l'ordre inverse les étapes de réaction mentionnées, de sorte que, sur les 37,8% en poids de départ, il y ait encore 3,8 à 13,2% en poids de groupes isocyanates libres par rapport au poids du polyisocyanate modifié, compte non tenu du poids de l'agent bloquant présent.

2. Utilisation du polyisocyanate obtenu selon la revendication 1 comme composant isocyanate dans les vernis en poudre de polyuréthannes à deux composants thermoréticulables.